# EUROPÄISCHE PATENTSCHRIFT

(11) **EP 0 461 489 B1**
(45) Veröffentlichungstag und Bekanntmachung des Hinweises auf die Patenterteilung: **26.07.1995**
(21) Anmeldenummer: 91108922.5
(22) Anmeldetag: 31.05.1991
(51) Int. Cl.: C08F 222/06

(54) **Verfahren zur Herstellung von Copolymerisaten aus Maleinsäuremonoalkylestern oder Maleinsäure und deren Salzen und Vinylalkylethern**
Process for preparation of copolymers of maleic acid monoalkyl esters or maleic acid and their salts and vinylalkyl ethers
Procédé de préparation de copolymères d'esters monoalcoyle d'acide maléique ou d'acide maléique et de leurs sels et d'éthers d'alcoyle et de vinyle

(30) Priorität: 13.06.1990 DE 4018874
(43) Veröffentlichungstag der Anmeldung: 18.12.1991
(73) Patentinhaber: BASF Aktiengesellschaft, 67063 Ludwigshafen (DE)
(72) Erfinder: Meyer, Harald, Dr., W-6705 Deidesheim (DE); Sanner, Axel, Dr., W-6710 Frankenthal (DE); Raubenheimer, Hans-Juergen, W-6834 Ketsch (DE); Frosch, Franz, Dr., W-6702 Bad Duerkheim (DE)

(56) Entgegenhaltungen:
- EP-A- 0 276 464
- DE-A- 3 733 158
- US-A- 3 499 876

## Beschreibung

Die vorliegende Erfindung betrifft ein verbessertes Verfahren zur Herstellung von Copolymerisaten aus Maleinsäuremonoalkylestern oder Maleinsäure und deren Salzen und Vinylalkylethern durch radikalische Copolymerisation von Maleinsäureanhydrid und Vinylalkylethern und anschließende Umsetzung mit Alkanol oder Wasser und gegebenenfalls Metallhydroxiden und/oder -oxiden, wobei in jeder Phase der Polymerisation die Vinylalkylether-Komponente im überschuß im Reaktionsgemisch vorliegt, die radikalische Copolymerisation in Aceton erfolgt und das Aceton während oder nach der Veresterung bzw. Hydrolyse destillativ entfernt wird.

In den Patentschriften US-A 2 694 697 (1) und US-A 3 499 876 (2) werden Polymerisationsverfahren für die Herstellung von Copolymerisaten aus Maleinsäureanhydrid und Vinylalkylethern beschrieben, bei denen u.a. Aceton als Lösungsmittel und als Radikalinitiatoren Azobisisobutyronitril, Dimethylazoisobutyrat, Benzoylperoxid, Laurylperoxid, Caprylylperoxid, Acetylperoxid, Acetylbenzoylperoxid oder Di-tert.-butylperoxid eingesetzt werden.

Die DE-A 37 33 158 (3), deren Anmeldetag später als die Veröffentlichungsdaten von (1) und (2) liegt, betrifft ein Verfahren zur Herstellung von Copolymerisaten aus Maleinsäuremonoalkylestern und Vinylalkylethern durch radikalische Copolymerisation von Maleinsäureanhydrid und Vinylalkylethern in Aceton und anschließende Umsetzung mit Alkanol, wobei als Polymerisationsinitiatoren insbesondere Azoverbindungen empfohlen werden; Peroxy-Gruppen enthaltende Initiatoren werden ausdrücklich als im allgemeinen weniger geeignet bezeichnet, weil mit ihnen nur schwer ein unmittelbares und gleichmäßiges Einsetzen der Polymerisation zu erreichen sei.

Da Maleinsäure- bzw. Maleinsäuremonoalkylester-Vinylalkylether-Copolymerisate hauptsächlich in kosmetischen Zubereitungen, insbesondere in der Haarkosmetik, eingesetzt werden, sind größere Gehalte an Maleinsäureanhydrid-Restmonomer oder dessen Hydrolyseprodukten wegen deren toxischer Eigenschaften nicht erwünscht. Die gemäß (3) hergestellten Copolymerisat-Lösungen weisen nach dem Polymerisationsschritt immer noch Maleinsäureanhydrid-Gehalte von 0,045 bis 0,4 Gew.-% auf.

In der EP-A 0 276 464 wird die Herstellung von Copolymerisaten der Maleinsäure durch radikalische Copolymerisation in einem aromatischen Kohlenwasserstoff beschrieben, wobei zur Senkung des Restmonomerengehalts eine Nachpolymerisation in Gegenwart eines Peroxiesters empfohlen wird, und die so hergestellten Copolymerisate den jeweils verwendeten Kohlenwasserstoff in gebundener Form enthalten.

Aufgabe der vorliegenden Erfindung war es daher, ein Verfahren zur Herstellung dieser Copolymerisate bereitzustellen, welches Produkte mit noch niedrigeren Gehalten an monomerem Maleinsäureanhydrid bzw. dessen Hydrolyseprodukten liefert.

Demgemäß wurde das eingangs definierte Verfahren gefunden, welches dadurch gekennzeichnet ist, daß man als Polymerisationsinitiatoren Carbonsäureperoxyester, die sich von Carbonsäuren mit mindestens 8 C-Atomen ableiten, verwendet.

In einer bevorzugten Ausführungsform werden als Polymerisationsinitiatoren Alkyl- oder Aralkylcarbonsäureperoxyester, die sich von Carbonsäuren mit 8 bis 10 C-Atomen ableiten, eingesetzt. Beispiele hierfür sind Cumylperneodecanoat, tert.-Butylperneodecanoat, tert.-Amylperneodecanoat, tert.-Butylperneooctanoat, tert.-Butylper-2-ethylhexanoat, tert.-Butylperisononanoat und tert.-Butylper-3,5,5-trimethylhexanoat. Die besten Ergebnisse erzielt man mit tert.-Butylperneodecanoat.

Die Carbonsäureperoxyester werden in den für Polymerisationsinitiatoren üblichen Mengen verwendet, beispielsweise 0,5 bis 3 Gew.-%, bezogen auf eingesetztes Maleinsäureanhydrid.

Die Polymerisation kann bei Normaldruck oder erhöhtem Druck, beispielsweise in einem Autoklaven, durchgeführt werden. Der Druck beträgt hierbei üblicherweise bis zu 5 bar, vorzugsweise bis zu 3 bar. Die Polymerisationstemperatur sollte zwischen 30 und 100°C, vorzugsweise zwischen 40 und 70°C, liegen.

Es ist nicht empfehlenswert, das Reaktionsgefäß mit der Gesamtmenge der Monomeren zu befüllen, da so die Reaktion leicht außer Kontrolle geraten kann. Zweckmäßigerweise wird ein kleiner Teil der Monomeren vorgelegt und der Rest innerhalb mehrerer Stunden zudosiert. Das Maleinsäureanhydrid kann hierbei in flüssiger Form oder als Lösung in Aceton eingebracht werden. Der Vinylalkylether wird in der Regel in flüssiger Form zugegeben. Im Falle von gasförmigen Vinylalkylethern empfiehlt es sich eine Einleitung des Gasstromes unterhalb der Oberfläche des Reaktionsgemisches.

Geeignete Vinylalkylether sind vor allem solche mit 1 bis 18 C-Atomen in der Alkylgruppe, z.B. Methylvinylether, Ethylvinylether, Butylvinylether, Isobutylvinylether, 2-Ethylhexylvinylether, Dodecylvinylether und Octadecylvinylether. Insbesondere eignen sich davon Vinylalkylether mit 1 bis 4 C-Atomen in der Alkylgruppe. Besonders bevorzugt wird Methylvinylether. Es können auch Mischungen verschiedener Vinylalkylether eingesetzt werden.

Zur Erzielung eines vollständigen oder nahezu vollständigen Umsatzes des Maleinsäureanhydrids ist es auch von Bedeutung, daß in jeder Phase der Polymerisation die Vinylalkylether-Komponente im überschuß im Reaktionsgemisch vorliegt. Der gesamte Vinylalkylether-Überschuß kann bis zu 50 mol-% betragen.

Das verwendete Aceton kann geringe Mengen an anderen Lösungsmitteln und an Wasser enthalten, ohne daß Beeinträchtigungen der Polymerisation zu beobachten sind. Der Wassergehalt von technischem Aceton, der etwa 0,3 Gew.-% beträgt, wirkt nicht störend.

Die Konzentration des gebildeten Polymeren in der acetonischen Lösung kann je nach Molekulargewicht und Struktur bis zu 70 Gew.-% betragen. Bevorzugt ist ein Bereich von 25 bis 60 Gew.-%. Nach Ende der Polymerisation wird zweckmäßigerweise ein Teil des Acetons abdestilliert, wobei bei Verwendung niedrig siedender Vinylalkylether auch der eingesetzte Vinylalkyletherüberschuß mitentfernt wird. Je nach Struktur und Molekulargewicht des Polymerisates kann bis zu einer Konzentration von 80 Gew.-% eingeengt werden, vorzugsweise auf 50 bis 70 Gew.-%.

Die destillative Entfernung der Gesamtmenge des Acetons erfolgt zweckmäßigerweise während oder nach der Veresterung bzw. Hydrolyse bei Temperaturen bis zu 70°C, am günstigsten bei etwa 50 bis 70°C. Das Abdestillieren kann bei Normaldruck oder vorzugsweise im Vakuum, insbesondere bei etwa 300 bis 1000 mbar, erfolgen, wobei in jedem Fall die thermische Belastung des Produktes so gering wie möglich gehalten werden sollte.

Die Veresterung der erhaltenen Maleinsäureanhydrid-Vinylalkylether-Copolymerisate mit Alkanolen erfolgt normalerweise analog der in (3) beschriebenen Methode, nämlich durch Zugabe des Alkanols zu der hochviskosen Lösung. Bevorzugt werden C₁- bis C₄-Alkanole, insbesondere Methanol, Ethanol, Isopropanol, n-Propanol und n-Butanol. Soll das gleiche Alkanol später auch als Lösungsmittel im Endprodukt dienen, kann ein überschuß des Alkanols von 100 mol-% oder mehr zugegeben werden. Soll das Alkanol jedoch nur zur Veresterung und nicht als Lösungsmittel dienen, ist ein großer überschuß zu vermeiden; in diesem Fall ist ein Alkanol-überschuß von bis zu 5 mol-% zweckmäßig.

Das polymere Anhydrid ist beispielsweise in Ethanol nicht löslich. Bei Zugabe von Ethanol bildet sich daher aus der acetonischen Lösung eine zähe, heterogene Masse, die erst im Verlauf der Veresterung homogen und klar wird.

Die Veresterung erfolgt zweckmäßigerweise in Gegenwart eines hierfür üblichen Katalysators in den üblichen Mengen, insbesondere eines sauren Katalysators wie vor allem Schwefelsäure oder p-Toluolsulfonsäure.

Die Zugabe des Alkanols erfolgt zweckmäßigerweise bei Temperaturen von etwa 50 bis 70°C. Falls das gleiche Alkanol später auch als Lösungsmittel dient, kann unmittelbar anschließend mit der destillativen Entfernung des Acetons begonnen werden, auch wenn die Veresterung noch nicht abgeschlossen ist. Wichtig für die gewünschte helle Farbe des Endproduktes ist es, daß die Temperatur des Reaktionsgemisches während der Umsetzung 70°C nicht überschreitet. Hierfür empfiehlt es sich, das Aceton hierbei unter einem schwachen Vakuum von etwa 300 bis 1000 mbar abzudestillieren. Ist das Aceton bis auf einen geringen Restgehalt von weniger als 2 Gew.-%, vorzugsweise weniger als 0,5 Gew.-%, bezogen auf das gesamte Reaktionsgemisch, entfernt worden, kann die Temperatur zur Vervollständigung der Veresterung auf etwa 80 bis 100°C erhöht werden, ohne daß nachteilige Wirkungen auftreten.

Ist das Alkanol, mit dem verestert wird, nicht der gleiche Alkohol, der auch als Lösungsmittel dienen soll, wird nach Zugabe des Veresterungsalkanols zunächst bis zur vollständigen Veresterung bei etwa 50 bis 70°C gerührt und erst dann wie oben beschrieben mit der Destillation begonnen.

Die erhaltenen Maleinsäuremonoalkylester-Vinylalkylether-Copolymerisate werden normalerweise als alkoholische Lösungen weiterverwendet. Man kann aber auch durch Abdestillieren des Lösungsmittels Feststoffe herstellen.

Die Hydrolyse der erhaltenen Maleinsäureanhydrid-Vinylalkylether-Copolymerisate zu Maleinsäure-Vinylalkylether-Copolymerisaten erfolgt zweckmäßigerweise durch Zugabe von Wasser zu der acetonischen Anhydridlösung bei etwa 50 bis 70°C. Es kann ein großer Wasser-überschuß von 1000 mol-% oder mehr zugegeben werden.

Man kann unmittelbar anschließend mit der destillativen Entfernung des Acetons beginnen, bei Normaldruck oder vorzugsweise unter einem schwachen Vakuum von etwa 300 bis 1000 mbar, auch wenn die Hydrolyse noch nicht abgeschlossen ist. In das Reaktionsgemisch kann das gesamte Wasser zu Beginn der Hydrolyse zugegeben werden. Die Zugabe kann aber auch kontinuierlich oder je nach Bedarf absatzweise erfolgen. Ist das Aceton bis auf einen geringen Restgehalt von weniger als 1 Gew.-%, vorzugsweise weniger als 0,01 Gew.-%, bezogen auf das gesamte Reaktionsgemisch, entfernt, kann die Temperatur bis 100°C erhöht werden, um die Hydrolyse zu vervollständigen.

Die wäßrigen Lösungen der Maleinsäure-Vinylalkylether-Copolymerisate können nach den üblichen Verfahren bis zur Gewichtskonstanz zu Feststoffen entwässert werden.

Eine teilweise oder vollständige Neutralisation der Maleinsäure-Vinylalkylether-Copolymerisate, gleichgültig ob sie als Lösungen oder Feststoffe vorliegt, kann gegebenenfalls mit Metallhydroxiden und/oder -oxiden erfolgen. Es kann von Vorteil sein, wenn man die Hydroxide oder Oxide vor Abschluß der Hydrolyse der Reaktionsmischung zugibt. Der Zeitpunkt kann aber auch von Beginn bis Ende der Hydrolyse variieren.

Die erhaltenen wäßrigen Lösungen der Metallsalze der Maleinsäure-Vinylalkylether-Copolymerisate können gewünschtenfalls nach den üblichen Verfahren bis zur Gewichtskonstanz zu Feststoffen entwässert werden.

Als Metallhydroxide, welche bei der Neutralisation besser als Metalloxide einsetzbar sind, dienen vor allem Alkalimetall- und Erdalkalimetallhydroxide wie Natriumhydroxid, Kaliumhydroxid und Calciumhydroxid. Daneben können aber auch beispielsweise Erdalkalimetalloxide wie Calciumoxid oder Magnesiumoxid verwendet werden. Auch Mischungen der genannten Stoffe sind einsetzbar.

Gegenstand der vorliegenden Erfindung ist weiterhin die Verwendung der nach dem erfindungsgemäßen Verfahren erhaltenen Copolymerisate in kosmetischen Zubereitungen. Vor allem dienen diese Copolymerisate als filmbildende Harze in Haarfestigungsmitteln wie Haargelen, Haarfestigerlösungen, Haarschäumen und insbesondere Haarsprays.

Die nach dem erfindungsgemäßen Verfahren erhaltenen Copolymerisate zeichnen sich vor allem durch einen sehr niedrigen Gehalt an Maleinsäureanhydrid-Restomonomer bzw. dessen Hydrolyseprodukten aus. Der Gehalt an monomerem Maleinsäureanhydrid in der Reaktionslösung beträgt nach dem Polymerisationsschritt in der Regel höchstens 0,006 Gew.-%, meist 0,002 bis 0,005 Gew.-%, im Gegensatz zu 0,045 bis 0,4 Gew.-% des Standes der Technik.

Auch enthalten die nach dem erfindungsgemäßen Verfahren hergestellten Copolymerisate keine ebenfalls toxikologisch bedenklichen und außerdem zu Geruchsbelästigungen führenden Zerfallsprodukte solcher Radikalinitiatoren, z.B. Azoverbindungen, die bislang auf diesem Gebiet eingesetzt wurden. Die Zerfallsprodukte der erfindungsgemäß verwendeten Carbonsäureperoxyester sind toxikologisch unbedenklich und geruchlos.

Weiterhin sind die nach dem erfindungsgemäßen Verfahren hergestellten Copolymerisate farblos oder nur schwach gelblich gefärbt, was für ihr Verwendung in kosmetischen Zubereitungen ebenfalls von Bedeutung ist.

### Beispiele

Die in den nachfolgenden Beispielen hergestellten Copolymerisate wurden durch ihren K-Wert nach Fikentscher, Cellulosechemie, Bd. 13, S. 58 bis 64 (1932), gemessen - wenn nicht anders vermerkt - in Cyclohexanon bei einer Konzentration von 1 g/100 ml bei 25°C, charakterisiert; der K-Wert stellt ein Maß für das Molgewicht dar. Die Verfärbung der Copolymerisat-Lösungen wurde durch ihre Iodfarbzahl gemäß DIN 6162 charakterisiert.

### Beispiel 1

### Herstellung eines Maleinsäuremonoethylester-Vinylmethylether-Copolymerisates

Ein 160 l-Stahlrührbehälter, der mit Dosiervorrichtungen und automatischer Innentemperatursteuerung ausgestattet war, wurde durch Spülen mit Stickstoff sauerstofffrei gemacht. Dann wurden 7 l Aceton vorgelegt. Von einer Lösung von 20 kg Maleinsäureanhydrid in 28 kg Aceton, die als Zulauf 1 diente, wurden 3 l zugegeben. Zulauf 2 bestand aus 17,6 l Vinylmethylether. Hiervon wurden 1 l zugegeben. Danach wurde der Reaktionsbehälter unter Druck auf 60°C aufgeheizt. Bei 60°C wurde 1 l von Zulauf 3 zugegeben, der aus 300 g tert.-Butylperneodecanoat in 6 kg Aceton bestand. Es wurde 15 Minuten bei 60°C anpolymerisiert und dann wurden bei dieser Innentemperatur Zulauf 1 und Zulauf 2 in 4 h und Zulauf 3 in 5 h zugefahren. Der Druck lag hierbei zwischen 1 und 1,5 bar. Nach Ende der Polymerisation wurde vorsichtig entspannt und bei Normaldruck wurden ca. 15 l Aceton abdestilliert.

Man erhielt eine farblose Polymerisatlösung mit einer Iodfarbzahl von 1 und mit einem Feststoffgehalt von 52,3 Gew.-%. Der K-Wert des Polymerisates betrug 46,5. Der Gehalt der Lösung an monomerem Maleinsäureanhydrid betrug 0,005 Gew.-%.

Zu dieser Polymerisatlösung wurden bei 54°C innerhalb von 1 h 40 g p-Toluolsulfonsäure in 20 l Ethanol zugegeben, wobei eine zähe, breiige, heterogene Masse entstand, die im Verlauf von 4 h Rühren bei dieser Temperatur langsam homogener wurde. Nach diesen 4 h wurde begonnen, bei ca. 500 mbar Lösungsmittel abzudestillieren, wobei immer dann, wenn die Grenze der Rührbarkeit erreicht war, 10 l Ethanol zugesetzt wurden (insgesamt fünfmal). Die Innentemperatur wurde bei 50 bis 55°C gehalten. Insgesamt wurden ca. 70 l Aceton-Ethanol-Gemisch abdestilliert. Nach dem Abkühlen wurde der Feststoffgehalt der Lösung mit Ethanol auf 49,5 Gew.-% eingestellt. Das erhaltene Copolymerisat hatte einen K-Wert von 39,6. Die Säurezahl betrug 145 mg KOH/g. Die Lösung war farblos (Iodfarbzahl < 1) und hatte einen esterartigen Geruch.

### Beispiel 2

### Herstellung eines Maleinsäuremonobutylester-Vinylmethylether-Copolymerisates

Ein 160 l-Stahlrührbehälter, der mit Dosiervorrichtungen und automatischer Innentemperatursteuerung ausgestattet war, wurde durch Spülen mit Stickstoff sauerstofffrei gemacht. Dann wurden 7 l Aceton vorgelegt. Von einer Lösung von 20 kg Maleinsäureanhydrid in 28 kg Aceton, die als Zulauf 1 diente, wurden 3 l zugegeben. Zulauf 2 bestand aus 17,6 l Vinylmethylether. Hiervon wurden 1 l zugegeben. Danach wurde der Reaktionsbehälter unter Druck auf 65°C aufgeheizt. Bei 65°C wurde 1 l von Zulauf 3 zugegeben, der aus 300 g tert.-Butylperneodecanoat in 6 kg Aceton bestand. Es wurde 15 Minuten bei 65°C anpolymerisiert und dann wurden bei dieser Innentemperatur Zulauf 1 und Zulauf 2 in 4 h und Zulauf 3 in 5 h zugefahren. Der Druck lag hierbei zwischen 1,5 und 2 bar. Nach Ende der Polymerisation wurde vorsichtig entspannt und bei Normaldruck wurden ca. 15 l Aceton abdestilliert.

Man erhielt eine farblose Polymerisatlösung mit einer Iodfarbzahl von < 1 und mit einem Feststoffgehalt von 54,1 Gew.-%. Der K-Wert des Polymerisates betrug 39,1. Der Gehalt der Lösung an monomerem Maleinsäureanhydrid betrug 0,004 Gew.-%.

Zu dieser Polymerisatlösung wurden innerhalb von 30 Minuten bei 53°C 15,9 kg n-Butanol und 40 g p-Toluolsulfonsäure gegeben. Dann wurde unter Druck 4 h auf 110°C erhitzt. Nach Abkühlen auf 60°C wurde bei ca. 500 mbar Aceton abdestilliert und fünfmal Ethanol in 10 l-Portionen zugegeben, so daß die Mischung stets rührbar blieb. Insgesamt wurde 75 l Aceton-Ethanol-Gemisch abdestilliert, wobei die Temperatur in der Lösung zwischen 50 und 55°C lag. Nach dem Abkühlen wurde der Feststoffgehalt mit Ethanol auf 52,0 Gew.-% eingestellt. Das erhaltene Copolymerisat hatte einen K-Wert von 40,9. Die Säurezahl der Lösung betrug 133 mg KOH/g. Die Lösung hatte eine Iodfarbzahl von 1 und einen esterartigen Geruch. Der Butanolgehalt betrug 4,9 Gew.-%.

### Beispiel 3

### Herstellung eines Maleinsäuremonoethylester-Vinylethylether-Copolymerisates

In einem 1 l-Glasrührbehälter, der mit Rührer, Rückflußkühler und Dosiereinrichtung versehen war, wurden unter Stickstoff 100 g Aceton vorgelegt. Aus 120 g Maleinsäureanhydrid und 130 g Aceton wurde eine Lösung bereitet, die als Zulauf 1 diente. 97 g Vinylethylether dienten als Zulauf 2. Eine Lösung von 2,1 g tert.-Butylperneodecanoat in 40 g Aceton bildete Zulauf 3. Von Zulauf 1 wurden 50 ml, von Zulauf 2 30 ml und von Zulauf 3 6 ml in die Vorlage gegeben und danach wurde zum Sieden erwärmt. Nach 10 Minuten Sieden wurden Zulauf 1 und Zulauf 2 gleichzeitig innerhalb von 3 Stunden und Zulauf 3 in 4 Stunden zudosiert, wobei ständig ein leichter Rückfluß aufrechterhalten wurde. Nach beendeter Zugabe des Zulaufes 3 wurde ca. 1 h bei leichtem Rückfluß nachpolymerisiert. Anschließend wurden ca. 80 ml Aceton abdestilliert.

Man erhielt eine farblose Lösung (Iodfarbzahl < 1) mit einem Feststoffgehalt von 53,4 Gew.-%. Der K-Wert des Polymerisates betrug 52,3. Der Gehalt der Lösung an monomerem Maleinsäureanhydrid betrug 0,005 Gew.-%.

Zu dieser Lösung wurden innerhalb von 30 Minuten 0,2 g p-Toluolsulfonsäure, gelöst in 130 g Ethanol, gegeben und es wurde 4 Stunden bei 65°C gerührt. Dann wurde bei ca. 500 mbar Lösungsmittel abdestilliert, wobei nach und nach 150 g Ethanol in 5 Portionen zugegeben wurde, um die Mischung rührbar zu halten. Insgesamt wurden 270 g Lösungsmittel abdestilliert. Nach Ende der Destillation erhielt man eine farblose Lösung mit einem Feststoffgehalt von 49,4 Gew.-% und einer Säurezahl von 140 mg KOH/g. Der K-Wert des Polymerisates betrug 44,3. Die Iodfarbzahl der Lösung betrug < 1.

### Beispiel 4

### Herstellung eines Natriummaleinat-Vinylmethylether-Copolymerisates

In einem 2 l-Glasrührbehälter, der mit Ankerrührer, Rückflußkühler und Dosiereinrichtung versehen war, wurden unter Stickstoff 155 g Aceton vorgelegt. Aus 300 g Maleinsäureanhydrid und 420 g Aceton wurde eine Lösung bereitet, die als Zulauf 1 diente. 264 ml Vinylmethylether dienten als Zulauf 2. Eine Lösung von 4,5 g tert.-Butylperneodecanoat in 90 g Aceton bildete Zulauf 3. Von Zulauf 1 wurden 45 ml, von Zulauf 2 und von Zulauf 3 jeweils 15 ml in die Vorlage gegeben und danach wurde zum Sieden erwärmt. Nach 15 Minuten Sieden wurden Zulauf 1 und Zulauf 2 in 4 Stunden und Zulauf 3 in 5 Stunden zudosiert, wobei ständig ein Rückfluß aufrechterhalten wurde. Nach beendeter Zugabe des Zulaufes 3 wurde 2 Stunden bei Siedetemperatur nachpolymerisiert. Anschließend wurden 150 ml Aceton abdestilliert.

Man erhielt eine klare, farblose Lösung (Iodfarbzahl < 1) mit einem Feststoffgehalt von 47,8 Gew.-%. Der K-Wert betrug 58,3. Der Gehalt der Lösung an monomerem Maleinsäureanhydrid betrug 0,006 Gew.-%.

Zu dieser Lösung wurden bei 56°C innerhalb von 1 Stunde 60 g Wasser gegeben und es wurde 1 Stunde bei dieser Temperatur gerührt. Dann wurde bei 800 mbar Lösungsmittel abdestilliert, wobei nach und nach 550 g Wasser portionsweise zugegeben wurde. Insgesamt wurden ca. 800 ml Lösungsmittel abdestilliert. Nach Ende der Destillation erhielt man eine leicht gelbliche Lösung mit einem Feststoffgehalt von 35,4 Gew.-%. Der K-Wert (1 Gew.-% in Wasser bei 25°C) betrug 61,6.

Zu dieser Lösung wurden bei 25°C 360 g 50 gew.-%ige wäßrige Natriumhydroxidlösung gegeben und es wurde 2 Stunden gerührt. Die abgekühlte Lösung hatte einem Feststoffgehalt von 38,0 Gew.-% und einen pH-Wert von ca. 9. Der K-Wert des Polymeren betrug 104,3 (1 Gew.-% in Wasser bei 25°C). Der Neutralisationsgrad der Carboxylgruppen betrug 73 %.

## Patentansprüche (Patentansprüche für folgende(n) Vertragsstaat(en): DE, FR, GB, IT, NL)

1. Verfahren zur Herstellung von Copolymerisaten aus Maleinsäuremonoalkylestern oder Maleinsäure und deren Salzen und Vinylalkylethern durch radikalische Copolymerisation von Maleinsäureanhydrid und Vinylalkylethern und anschließende Umsetzung mit Alkanol oder Wasser und gegebenenfalls Metallhydroxiden und/oder -oxiden, wobei in jeder Phase der Polymerisation die Vinylalkylether-Komponente im Überschuß im Reaktionsgemisch vorliegt, die radikalische Copolymerisation in Aceton erfolgt und das Aceton während oder nach der Veresterung bzw. Hydrolyse destillativ entfernt wird, dadurch gekennzeichnet, daß man als Polymerisationsinitiatoren Carbonsäureperoxyester, die sich von Carbonsäuren mit mindestens 8 C-Atomen ableiten, verwendet.

2. Verfahren nach Anspruch 1, dadurch gekennzeichnet, daß man als Polymerisationsinitiatoren Alkyl- oder Aralkylcarbonsäureperoxyester, die sich von Carbonsäuren mit 8 bis 10 C-Atomen ableiten, verwendet.

3. Verfahren nach Anspruch 2, dadurch gekennzeichnet, daß man als Polymerisationsinitiator tert.-Butylperneodecanoat verwendet.

4. Verfahren nach den Ansprüchen 1 bis 3, dadurch gekennzeichnet, daß man die radikalische Copolymerisation bei Normaldruck oder einem Druck bis zu 5 bar und bei Temperaturen zwischen 30 und 100°C durchführt.

5. Verfahren nach den Ansprüchen 1 bis 4, dadurch gekennzeichnet, daß man einen Alkylvinylether mit 1 bis 18 C-Atomen in der Alkylgruppe einsetzt.

6. Verfahren nach den Ansprüchen 1 bis 5, dadurch gekennzeichnet, daß man das Aceton während oder nach der Veresterung bzw. Hydrolyse bei Temperaturen bis zu 70°C destillativ entfernt.

7. Verfahren nach den Ansprüchen 1 bis 6, dadurch gekennzeichnet, daß man die Umsetzung der Copolymerisate aus Maleinsäureanhydrid und Vinylalkylethern mit einem C₁- bis C₄-Alkanol durchführt.

8. Verfahren nach den Ansprüchen 1 bis 6, dadurch gekennzeichnet, daß man die Umsetzung der Copolymerisate aus Maleinsäureanhydrid und Vinylalkylethern mit Wasser und gegebenenfalls einem Alkalimetall- und/oder Erdalkalimetallhydroxid durchführt.

## Patentansprüche (Patentansprüche für folgende(n) Vertragsstaat(en): ES)

1. Verfahren zur Herstellung von Copolymerisaten aus Maleinsäuremonoalkylestern oder Maleinsäure und deren Salzen und Vinylalkylethern durch radikalische Copolymerisation von Maleinsäureanhydrid und Vinylalkylethern und anschließende Umsetzung mit Alkanol oder Wasser und gegebenenfalls Metallhydroxiden und/oder -oxiden, wobei in jeder Phase der Polymerisation die Vinylalkylether-Komponente im überschuß im Reaktionsgemisch vorliegt, die radikalische Copolymerisation in Aceton erfolgt und das Aceton während oder nach der Veresterung bzw. Hydrolyse destillativ entfernt wird, dadurch gekennzeichnet, daß man als Polymerisationsinitiatoren Carbonsäureperoxyester, die sich von Carbonsäuren mit mindestens 8 C-Atomen ableiten, verwendet.

2. Verfahren nach Anspruch 1, dadurch gekennzeichnet, daß man als Polymerisationsinitiatoren Alkyl- oder Aralkylcarbonsäureperoxyester, die sich von Carbonsäuren mit 8 bis 10 C-Atomen ableiten, verwendet.

3. Verfahren nach Anspruch 2, dadurch gekennzeichnet, daß man als Polymerisationsinitiator tert.-Butylperneodecanoat verwendet.

4. Verfahren nach den Ansprüchen 1 bis 3, dadurch gekennzeichnet, daß man die radikalische Copolymerisation bei Normaldruck oder einem Druck bis zu 5 bar und bei Temperaturen zwischen 30 und 100°C durchführt.

5. Verfahren nach den Ansprüchen 1 bis 4, dadurch gekennzeichnet, daß man einen Alkylvinylether mit 1 bis 18 C-Atomen in der Alkylgruppe einsetzt.

6. Verfahren nach den Ansprüchen 1 bis 5, dadurch gekennzeichnet, daß man das Aceton während oder nach der Veresterung bzw. Hydrolyse bei Temperaturen bis zu 70°C destillativ entfernt.

7. Verfahren nach den Ansprüchen 1 bis 6, dadurch gekennzeichnet, daß man die Umsetzung der Copolymerisate aus Maleinsäureanhydrid und Vinylalkylethern mit einem C₁- bis C₄-Alkanol durchführt.

8. Verfahren nach den Ansprüchen 1 bis 6, dadurch gekennzeichnet, daß man die Umsetzung der Copolymerisate aus Maleinsäureanhydrid und Vinylalkylethern mit Wasser und gegebenenfalls einem Alkalimetall- und/oder Erdalkalimetallhydroxid durchführt.

## Claims (Claims for the following Contracting State(s): DE, FR, GB, IT, NL)

1. A process for preparing a copolymer of a monoalkyl maleate, maleic acid or a salt thereof and a vinyl alkyl ether by free radical copolymerization of maleic anhydride and a vinyl alkyl ether and subsequent reaction with an alkanol or water and optionally a metal hydroxide or oxide, wherein at every stage of the polymerization the vinyl alkyl ether component is present in the reaction mixture in excess, the free radical copolymerization is carried out in acetone and the acetone is removed by distillation during or after the esterification or hydrolysis, which comprises using a peroxyester of a carboxylic acid of 8 or more carbon atoms as polymerization initiator.

2. A process as claimed in claim 1, wherein the polymerization initiator used is a peroxyalkyl or peroxyaralkyl ester of a carboxylic acid of from 8 to 10 carbon atoms.

3. A process as claimed in claim 2, wherein the polymerization initiator used is tert-butyl perneodecanoate.

4. A process as claimed in claim 1 or 2 or 3, wherein the free radical copolymerization is carried out under atmospheric pressure or under a pressure of up to 5 bar and at from 30 to 100°C.

5. A process as claimed in claim 1 or 2 or 3 or 4, wherein the alkyl vinyl ether used has from 1 to 18 carbon atoms in the alkyl group.

6. A process as claimed in claim 1 or 2 or 3 or 4 or 5, wherein the distillative removal of the acetone during or after the esterification or hydrolysis is effected at up to 70°C.

7. A process as claimed in claim 1 or 2 or 3 or 4 or 5 or 6, wherein the copolymer of maleic anhydride and a vinyl alkyl ether is reacted with a C₁-C₄-alkanol.

8. A process as claimed in claim 1 or 2 or 3 or 4 or 5 or 6, wherein the copolymer of maleic anhydride and a vinyl alkyl ether is reacted with water and optionally an alkali metal hydroxide or an alkaline earth metal hydroxide.

## Claims (Claims for the following Contracting State(s): ES)

1. A process for preparing a copolymer of a monoalkyl maleate, maleic acid or a salt thereof and a vinyl alkyl ether by free radical copolymerization of maleic anhydride and a vinyl alkyl ether and subsequent reaction with an alkanol or water and optionally a metal hydroxide or oxide, wherein at every stage of the polymerization the vinyl alkyl ether component is present in the reaction mixture in excess, the free radical copolymerization is carried out in acetone and the acetone is removed by distillation during or after the esterification or hydrolysis, which comprises using a peroxyester of a carboxylic acid of 8 or more carbon atoms as polymerization initiator.

2. A process as claimed in claim 1, wherein the polymerization initiator used is a peroxyalkyl or peroxyaralkyl ester of a carboxylic acid of from 8 to 10 carbon atoms.

3. A process as claimed in claim 2, wherein the polymerization initiator used is tert-butyl perneodecanoate.

4. A process as claimed in claim 1 or 2 or 3, wherein the free radical copolymerization is carried out under atmospheric pressure or under a pressure of up to 5 bar and at from 30 to 100°C.

5. A process as claimed in claim 1 or 2 or 3 or 4, wherein the alkyl vinyl ether used has from 1 to 18 carbon atoms in the alkyl group.

6. A process as claimed in claim 1 or 2 or 3 or 4 or 5, wherein the distillative removal of the acetone during or after the esterification or hydrolysis is effected at up to 70°C.

7. A process as claimed in claim 1 or 2 or 3 or 4 or 5 or 6, wherein the copolymer of maleic anhydride and a vinyl alkyl ether is reacted with a C₁-C₄-alkanol.

8. A process as claimed in claim 1 or 2 or 3 or 4 or 5 or 6, wherein the copolymer of maleic anhydride and a vinyl alkyl ether is reacted with water and optionally an alkali metal hydroxide or an alkaline earth metal hydroxide.

## Revendications (Revendications pour l'(les) Etat(s) contractant(s) suivant(s): DE, FR, GB, IT, NL)

1. Procédé de préparation de copolymères d'esters monoalkyliques d'acide maléique ou d'acide maléique et de sels de celui-ci et d'oxydes d'alkyle et de vinyle par copolymérisation radicalaire d'anhydride maléique et d'oxydes d'alkyle et de vinyle, suivie d'une réaction avec un alcanol ou de l'eau et éventuellement avec des hydroxydes et/ou des oxydes métalliques, le composant oxyde d'alkyle et de vinyle étant présent en excès dans le mélange réactionnel dans chaque phase de la polymérisation, la copolymérisation radicalaire étant effectuée dans de l'acétone et l'acétone étant éliminée par distillation pendant ou après l'estérification ou l'hydrolyse, caractérisé en ce que l'on utilise, comme amorceurs de polymérisation, des peroxyesters d'acide carboxylique qui dérivent d'acides carboxyliques renfermant au moins 8 atomes de carbone.

2. Procédé selon la revendication 1, caractérisé en ce que l'on utilise, comme amorceurs de polymérisation, des peroxyesters alkyliques ou aralkyliques d'acide carboxylique qui dérivent d'acides carboxyliques à 8-10 atomes de carbone.

3. Procédé selon la revendication 2, caractérisé en ce que l'on utilise, comme amorceur de polymérisation, du pernéodécanoate de tert.-butyle.

4. Procédé selon l'une quelconque des revendications 1 à 3, caractérisé en ce que l'on conduit la copolymérisation radicalaire sous la pression normale ou sous une pression allant jusqu'à 5 bar et à des températures comprises entre 30 et 100°C.

5. Procédé selon l'une quelconque des revendications 1 à 4, caractérisé en ce que l'on met en réaction un oxyde d'alkyle et de vinyle à 1-18 atomes de carbone dans le groupement alkyle.

6. Procédé selon l'une quelconque des revendications 1 à 5, caractérisé en ce que l'on élimine par distillation l'acétone, pendant ou après l'estérification ou l'hydrolyse, à des températures allant jusqu'à 70°C.

7. Procédé selon l'une quelconque des revendications 1 à 6, caractérisé en ce que l'on fait réagir les copolymères d'anhydride maléique et d'oxydes d'alkyle et de vinyle avec un alcanol en C₁-C₄.

8. Procédé selon l'une quelconque des revendications 1 à 6, caractérisé en ce que l'on fait réagir les copolymères d'anhydride maléique et d'oxydes d'alkyle et de vinyle avec de l'eau et éventuellement avec un hydroxyde de métal alcalin et/ou de métal alcalino-terreux.

## Revendications (Revendications pour l'(les) Etat(s) contractant(s) suivant(s): ES)

1. Procédé de préparation de copolymères d'esters monoalkyliques d'acide maléique ou d'acide maléique et de sels de celui-ci et d'oxydes d'alkyle et de vinyle par copolymérisation radicalaire d'anhydride maléique et d'oxydes d'alkyle et de vinyle, suivie d'une réaction avec un alcanol ou de l'eau et éventuellement avec des hydroxydes et/ou des oxydes métalliques, le composant oxyde d'alkyle et de vinyle étant présent en excès dans le mélange réactionnel dans chaque phase de la polymérisation, la copolymérisation radicalaire étant effectuée dans de l'acétone et l'acétone étant éliminée par distillation pendant ou après l'estérification ou l'hydrolyse, caractérisé en ce que l'on utilise, comme amorceurs de polymérisation, des peroxyesters d'acide carboxylique qui dérivent d'acides carboxyliques renfermant au moins 8 atomes de carbone.

2. Procédé selon la revendication 1, caractérisé en ce que l'on utilise, comme amorceurs de polymérisation, des peroxyesters alkyliques ou aralkyliques d'acide carboxylique qui dérivent d'acides carboxyliques à 8-10 atomes de carbone.

3. Procédé selon la revendication 2, caractérisé en ce que l'on utilise, comme amorceur de polymérisation, du pernéodécanoate de tert.-butyle.

4. Procédé selon l'une quelconque des revendications 1 à 3, caractérisé en ce que l'on conduit la copolymérisation radicalaire sous la pression normale ou sous une pression allant jusqu'à 5 bar et à des températures comprises entre 30 et 100°C.

5. Procédé selon l'une quelconque des revendications 1 à 4, caractérisé en ce que l'on met en réaction un oxyde d'alkyle et de vinyle à 1-18 atomes de carbone dans le groupement alkyle.

6. Procédé selon l'une quelconque des revendications 1 à 5, caractérisé en ce que l'on élimine par distillation l'acétone, pendant ou après l'estérification ou l'hydrolyse, à des températures allant jusqu'à 70°C.

7. Procédé selon l'une quelconque des revendications 1 à 6, caractérisé en ce que l'on fait réagir les copolymères d'anhydride maléique et d'oxydes d'alkyle et de vinyle avec un alcanol en C₁-C₄.

8. Procédé selon l'une quelconque des revendications 1 à 6, caractérisé en ce que l'on fait réagir les copolymères d'anhydride maléique et d'oxydes d'alkyle et de vinyle avec de l'eau et éventuellement avec un hydroxyde de métal alcalin et/ou de métal alcalino-terreux.
